(19) Europäisches Patentamt / European Patent Office / Office européen des brevets

(11) **EP 4 212 914 A1**

(12) **EUROPEAN PATENT APPLICATION**
published in accordance with Art. 153(4) EPC

(43) Date of publication:
**19.07.2023 Bulletin 2023/29**

(21) Application number: **21888911.1**

(22) Date of filing: **03.09.2021**

(51) International Patent Classification (IPC):
**G01T 1/161** (2006.01)    **G01T 1/20** (2006.01)
**H01L 31/0232** (2014.01)    **H01L 31/107** (2006.01)

(52) Cooperative Patent Classification (CPC):
**G01T 1/161; G01T 1/20; H01L 31/0232; H01L 31/107**

(86) International application number:
**PCT/JP2021/032498**

(87) International publication number:
**WO 2022/097358 (12.05.2022 Gazette 2022/19)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **04.11.2020 JP 2020184278**

(71) Applicant: **Hamamatsu Photonics K.K.**
**Hamamatsu-shi, Shizuoka 435-8558 (JP)**

(72) Inventors:
• **UENOYAMA Soh**
  **Hamamatsu-shi, Shizuoka 435-8558 (JP)**
• **OTA Ryosuke**
  **Hamamatsu-shi, Shizuoka 435-8558 (JP)**

(74) Representative: **Grünecker Patent- und Rechtsanwälte**
**PartG mbB**
**Leopoldstraße 4**
**80802 München (DE)**

(54) **LIGHT DETECTOR, RADIATION DETECTOR, AND PET DEVICE**

(57) A light detector includes a semiconductor light detection element having a plurality of light detection units disposed two-dimensionally and readout wirings and a plurality of metalenses disposed on a surface of the semiconductor light detection element. Each of the plurality of light detection units has an avalanche photodiode including a first semiconductor region and a second semiconductor region forming a PN junction with the first semiconductor region, and a quenching resistor including one end electrically connected to the second semiconductor region and another end electrically connected to the readout wiring. The plurality of metalenses are disposed two-dimensionally to overlap the plurality of light detection units, and converge light such that a convergence spot is located at a position which is within the first semiconductor region and which is separated by a predetermined distance from a boundary between the first semiconductor region and the second semiconductor region.

**Fig.10**

## Description

## Technical Field

The present disclosure relates to a light detector, a radiation detector, and a PET device.

## Background Art

A time-of-flight positron emission tomography (TOF-PET) device has a plurality of radiation detectors disposed in an annular shape, and each radiation detector includes a light emitting body (a scintillator, Cherenkov radiator, or the like) that emits light upon incidence of radiation and a light detector that detects the light emitted by the light emitting body. In order to acquire a high-quality image in a TOF-PET device, it is necessary to uniformize time from generation of electric charges to detection of the electric charges (hereinafter referred to as "response time") and to improve single photon time resolution in a light detector of each radiation detector.

Patent Literature 1 describes a light detection device that includes a silicon photo multiplier (SiPM) having a plurality of single photon avalanche diodes (SPADs) and a plurality of microlenses disposed on a surface of the SiPM. In the light detection device disclosed in Patent Literature 1, in order to uniformize response time of each SPAD, a generation position of electric charges in each SPAD is controlled in a direction parallel to a surface of an SiPM by a plurality of microlenses.

## Citation List

## Patent Literature

[Patent Literature 1] Japanese Unexamined Patent Publication No. 2020-115575

## Summary of Invention

## Technical Problem

Although the light detection device described in Patent Literature 1 is applied to each radiation detector of a TOF-PET device and a generation position of electric charges in each SPAD is controlled in a direction parallel to a surface of an SiPM by a plurality of microlenses, it cannot be said that response time can be sufficiently uniformized in each SPAD from the viewpoint of improving single-photon time resolution.

An object of the present disclosure is to provide a light detector capable of sufficiently uniformizing response time, and a radiation detector and a PET device including such a light detector.

## Solution to Problem

According to an aspect of the present disclosure, there is provided a light detector including: a semiconductor light detection element having a plurality of light detection units disposed two-dimensionally and readout wirings; and a plurality of metalenses disposed on a surface of the semiconductor light detection element, wherein each of the plurality of light detection units has an avalanche photodiode including a first semiconductor region of a first conductivity type and a second semiconductor region of a second conductivity type, the second semiconductor region located on a side of the surface with respect to the first semiconductor region and forming a PN junction with the first semiconductor region, and a quenching resistor including one end electrically connected to the second semiconductor region and another end electrically connected to the readout wiring, and wherein the plurality of metalenses are disposed two-dimensionally to overlap the plurality of light detection units when seen in a direction intersecting with the surface, and converge light such that a convergence spot is located at a position which is within the first semiconductor region of each of the plurality of light detection units and which is separated by a predetermined distance from a boundary between the first semiconductor region and the second semiconductor region in the direction intersecting with the surface.

In the light detector according to the aspect of the present disclosure, the metalenses converge the light such that the convergence spot is located at a position which is within the first semiconductor region of each of the plurality of light detection units and which is separated by a predetermined distance from the boundary between the first semiconductor region and the second semiconductor region in the direction intersecting with the surface. As a result, the depth of the portion in the first semiconductor region where electric charges are generated is uniformized among the plurality of light detection units. Therefore, according to the light detector of the aspect of the present disclosure, the response time can be sufficiently uniformized.

In the light detector according to the aspect of the present disclosure, when a thickness of the first semiconductor region in the direction intersecting with the surface is defined as t, the predetermined distance may be less than t/2. According to this, the portion where the electric charges are generated in the first semiconductor region is sufficiently close to the second semiconductor region, and thus it is possible to more reliably curb variations in response time.

In the light detector according to the aspect of the present disclosure, when a standard deviation of a Gaussian function in a case where a beam profile of the convergence spot in the direction intersecting with the surface is approximated to the Gaussian function is defined as $\sigma$, the predetermined distance may be $0\sigma$ or more and $3\sigma$ or less. According to this, the portion where the electric charges are generated in the first semiconductor region is sufficiently close to the second semiconductor region, and thus it is possible to more reliably curb variations in response time.

In the light detector according to the aspect of the present disclosure, when a wavelength of the light is defined as $\lambda$ ($\mu$m), each of the plurality of metalenses may have a numerical aperture of $((0.61)^2\pi\lambda)^{1/2}$ or more. According to this, the Rayleigh length of the light converged by the metalenses is sufficiently shortened to about 1 $\mu$m or less, and thus it is possible to more reliably curb variations in response time.

In the light detector according to the aspect of the present disclosure, the predetermined distance may be 0 $\mu$m or more and 3 $\mu$m or less. According to this, the portion where the electric charges are generated in the first semiconductor region is sufficiently close to the second semiconductor region, and thus it is possible to more reliably curb variations in response time.

The light detector according to the aspect of the present disclosure may further include a light transmitting substrate provided with the plurality of metalenses, wherein the plurality of metalenses may be disposed on the surface of the semiconductor light detection element via the light transmitting substrate. According to this, in manufacturing the light detector, for example, it is possible to improve the yield of the light detector by bonding the light transmitting substrate on which the plurality of metalenses are formed to the surface of the semiconductor light detection element. In addition, it is possible to make the light transmitting substrate function as a spacer between the metalenses and the light detection unit, and it is possible to reliably locate the convergence spot at a position separated by the predetermined distance from the boundary between the first semiconductor region and the second semiconductor region.

The light detector according to the aspect of the present disclosure may further include a wiring substrate disposed on a side opposite to the plurality of metalenses with respect to the semiconductor light detection element, wherein the semiconductor light detection element may be electrically and physically connected to the wiring substrate. According to this, an external wiring can be connected to the wiring substrate from a side opposite to the plurality of metalenses.

According to an aspect of the present disclosure, there is provided a radiation detector including: a light emitting body configured to emit light upon incidence of radiation; and the light detector described above, wherein the light emitting body is disposed on a side opposite to the semiconductor light detection element with respect to the plurality of metalenses.

According to the radiation detector of the aspect of the present disclosure, it is possible to sufficiently uniformize the response time in the light detector for the reasons described above.

According to an aspect of the present disclosure, there is provided a PET device including: a plurality of radiation detectors disposed in an annular shape, wherein each of the plurality of radiation detectors is the radiation detector described above.

According to the PET device of the aspect of the present disclosure, it is possible to sufficiently uniformize the response time in the plurality of light detectors for the reasons described above, and thus it is possible to acquire a high-quality image.

**Advantageous Effects of Invention**

According to the present disclosure, it is possible to provide a light detector capable of sufficiently uniformizing response time, and a radiation detector and a PET device including such a light detector.

**Brief Description of Drawings**

FIG. 1 is a configuration diagram of a PET device of an embodiment.
FIG. 2 is a configuration diagram of a radiation detection device shown in FIG. 1.
FIG. 3 is a side view of a radiation detector shown in FIG. 2.
FIG. 4 is a plan view of a semiconductor light detection element shown in FIG. 3.
FIG. 5 is a circuit diagram of a light detector shown in FIG. 3.
FIG. 6 is a plan view of a portion of the semiconductor light detection element shown in FIG. 4.
FIG. 7 is a cross-sectional view of a portion of the semiconductor light detection element shown in FIG. 4.
FIG. 8 is a bottom view of the semiconductor light detection element shown in FIG. 4.
FIG. 9 is a plan view of a portion of the light detector shown in FIG. 3.
FIG. 10 is a cross-sectional view of a portion of the light detector along line X-X shown in FIG. 9.
FIG. 11 is a diagram showing a convergence state of light shown in FIG. 10.
FIG. 12 is a graph showing time resolution of light detectors of an example and a comparative example.
FIG. 13 is a cross-sectional view of a portion of a light detector of a modification example.

**Description of Embodiments**

Hereinafter, an embodiment of the present disclosure will be described in detail with reference to the drawings. The same or corresponding parts in the drawings are denoted with the same reference signs, and repetitive description will be omitted.

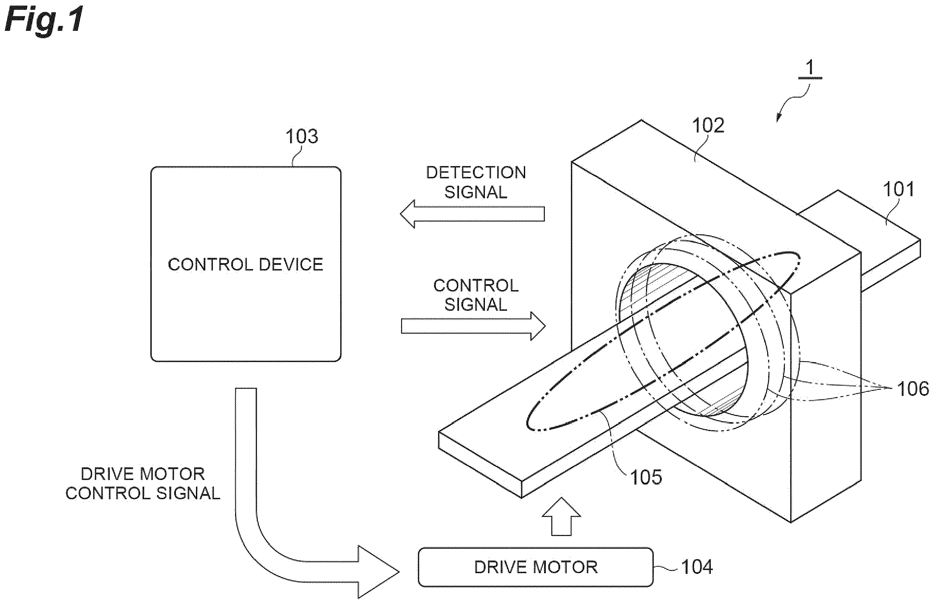

As shown in FIG. 1, the PET device 1 includes a cradle 101, a gantry 102, a control device 103, and a drive motor 104. The cradle 101 is disposed to pass through an opening in the gantry 102. A subject 105 is placed on the cradle 101. The control device 103 controls the drive motor 104 according to a drive motor control signal. As a result, the cradle 101 on which the subject 105 is placed moves, and the relative position of the subject 105 with respect to the opening of the gantry 102 changes. The drive motor 104 may be configured to move the gantry 102 or may be configured to move the cradle 101 and the gantry 102.

The gantry 102 has a plurality of radiation detection devices 106. The plurality of radiation detection devices 106 are disposed in a direction in which the opening of the gantry 102 penetrates. The radiation detection devices 106 surround the opening of the gantry 102. The control device 103 inputs a control signal for controlling each radiation detection device 106 to the gantry 102. The gantry 102 outputs a detection signal detected by each radiation detection device 106 to the control device 103.

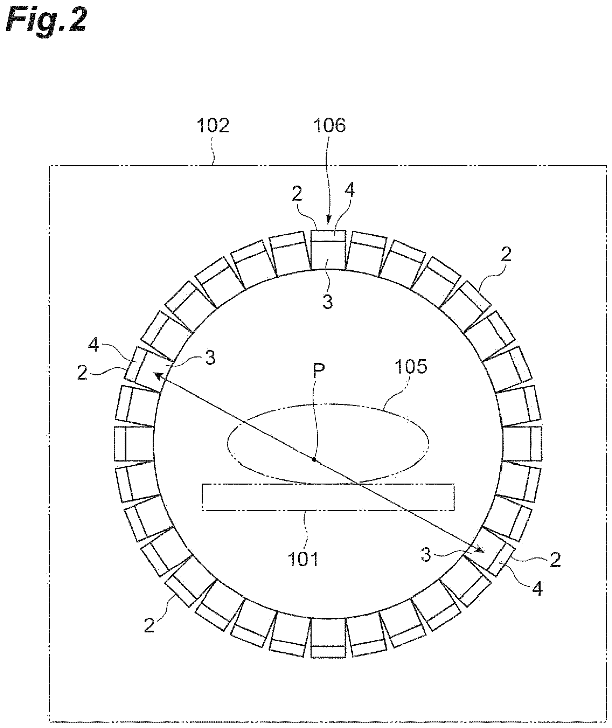

As shown in FIG. 2, the radiation detection device 106 includes a plurality of radiation detectors 2. The plurality of radiation detectors 2 are disposed in an annular shape to surround the opening of the gantry 102. The subject 105 is injected with a radioisotope (a positron emitting nuclide) that emits positrons. Positrons combine with negative electrons in the subject 105 to generate annihilation $\gamma$ rays. The annihilation $\gamma$ rays are emitted in opposite directions from a position P of the radioisotope within the subject 105. As a result, the annihilation $\gamma$ rays are detected by a pair of radiation detectors 2 facing each other with the position P interposed therebetween. The control device 103 identifies the position P on the basis of a time-of-flight difference of the annihilation $\gamma$ rays and generates an image (a tomographic image) regarding internal information of the subject 105. In other words, the PET device 1 is a TOF-PET device.

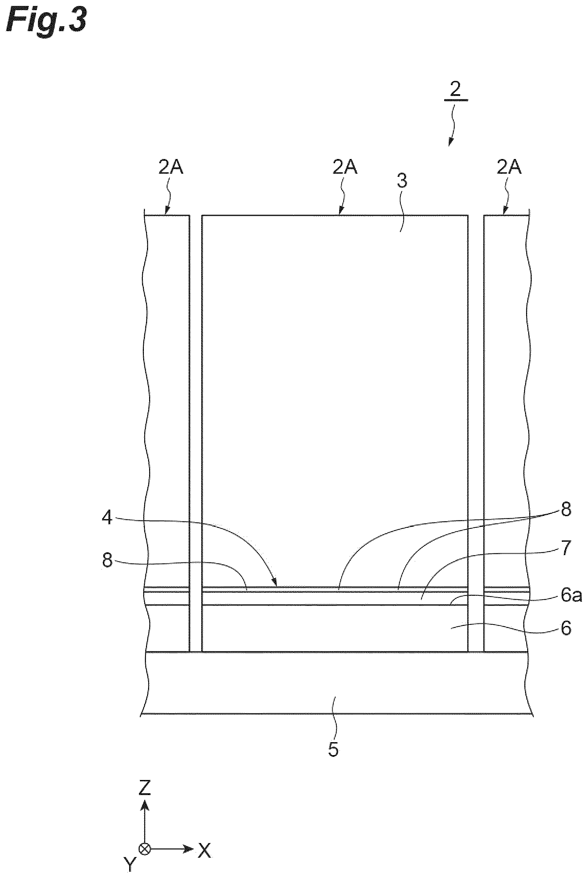

As shown in FIG. 3, the radiation detector 2 includes a plurality of radiation detection units 2A. The plurality of radiation detection units 2A are disposed in a matrix shape with an X-axis direction and a Y-axis direction as a row direction and a column direction. Each radiation detection unit 2A includes a scintillator (a light emitting body) 3 and a light detector 4. In FIG. 3, a Z-axis direction is a radial direction of an annulus (see FIG. 2) in which the plurality of radiation detectors 2 are disposed, an X-axis direction is a tangential direction of the annulus, and a Y-axis direction is a direction perpendicular to the Z-axis direction and the X-axis direction.

The scintillator 3 is disposed on the center side of the opening of the gantry 102 (hereinafter referred to as a "light incidence side") with respect to the light detector 4 (see FIG. 2). The scintillator 3 emits light (fluorescence) upon incidence of annihilation $\gamma$ rays. The scintillator 3 is formed of at least one material or a mixture of a plurality of materials selected from the group consisting of $Lu_{2-x}Y_xSiO_5{:}Ce$(LYSO), gadolinium aluminum gallium garnet (GAGG), NaI(Tl), Pr:LuAG, $LaBr_2$, $LaBr_3$, and $(Lu_xTb_{1-x-y}Ce_y)_3Al_5O_{12}$ (that is, LuTAG). In LuTAG, a composition ratio x is in the range of 0.5 to 1.5, and a composition ratio y is in the range of 0.01 to 0.15.

The light detector 4 detects the light emitted from the scintillator 3. The light detector 4 has a wiring substrate 5, a semiconductor light detection element 6, a light transmitting substrate 7, and a plurality of metalenses 8. The wiring substrate 5 is shared by a plurality of light detectors 4. The wiring substrate 5, the semiconductor light detection element 6, the light transmitting substrate 7, and the plurality of metalenses 8 are disposed in that order from a side opposite to the scintillator 3. That is, the scintillator 3 is disposed on a side opposite to the semiconductor light detection element 6 with respect to the plurality of metalenses 8. The scintillator 3 is bonded to the light detector 4 with a light transmitting adhesive.

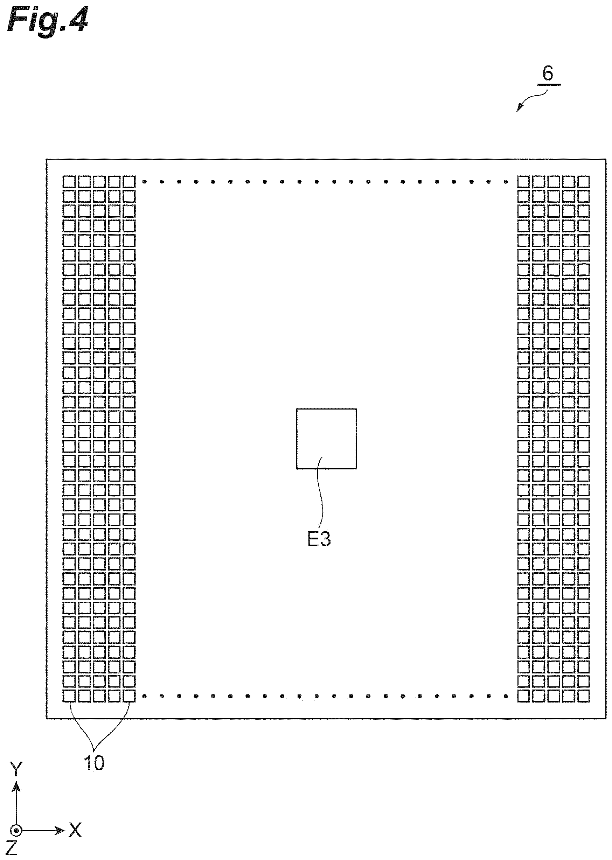

As shown in FIG. 4, the semiconductor light detection element 6 has a plurality of light detection units 10 disposed two-dimensionally and a common electrode E3. As an example, the semiconductor light detection element 6 has a rectangular shape when seen in the Z-axis direction, and the plurality of light detection units 10 are disposed in a matrix shape with the X-axis direction and the Y-axis direction as the row direction and the column direction. The common electrode E3 is located at the center of the semiconductor light detection element 6 when seen in the Z-axis direction. Electric charges generated in each light detection unit 10 are collected in the common electrode E3. That is, the semiconductor light detection element 6 is an SiPM having a plurality of SPADs (light detection units 10). In FIG. 4, the plurality of light detection units 10 are shown only in regions at both ends of the semiconductor light detection element 6, but the plurality of light detection units 10 are formed over the entire region of the semiconductor light detection element 6 except for the common electrode E3.

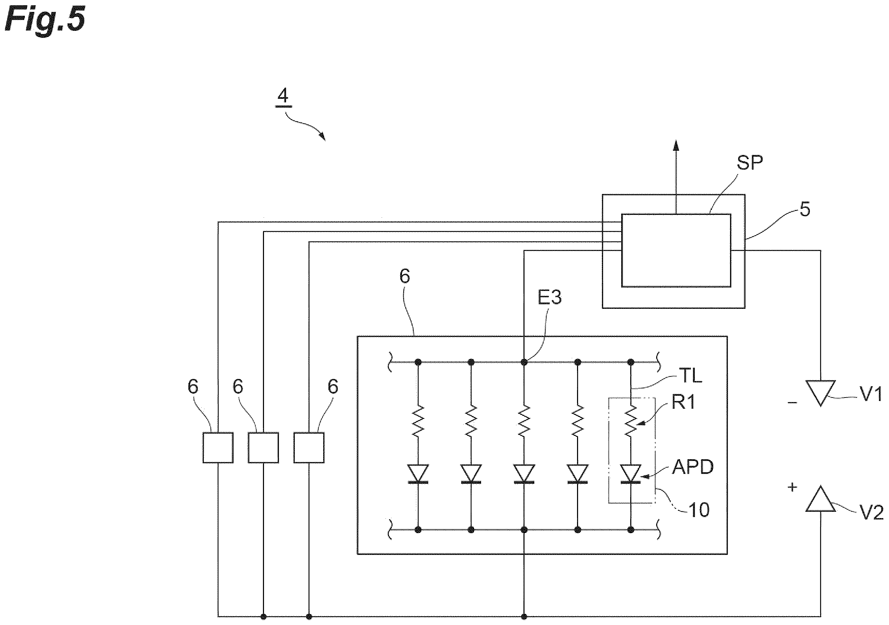

As shown in FIG. 5, each light detection unit 10 has an avalanche photodiode APD and a quenching resistor R1. One end of the quenching resistor R1 is electrically connected to the anode of the avalanche photodiode APD, and the other end of the quenching resistor R1 is electrically connected to the common electrode E3 via a readout wiring TL of the semiconductor light detection element 6. That is, the plurality of light detection units 10 are connected to each other in parallel, and in each light detection unit 10, the avalanche photodiode APD and the quenching resistor R1 are directly connected to each other. In the semiconductor light detection element 6, each avalanche photodiode APD is operated in Geiger mode. In the Geiger mode, a reverse voltage (a reverse bias voltage) higher than a breakdown voltage of the avalanche photodiode APD is applied to the avalanche photodiode APD. That is, a potential V1 is applied to the anode of the avalanche photodiode APD, and a potential V2 which is positive with respect to the potential V1 is applied to the cathode of the avalanche photodiode APD. The polarities of these potentials are relative, and for example, one of the potentials may be a ground potential.

The wiring substrate 5 is provided with a signal processing unit SP. The signal processing unit SP processes a signal output from each semiconductor light detection element 6 using each semiconductor light detection element 6 as a channel. The signal processing unit SP outputs the processed signal (the detection signal) to the control device 103 (see FIG. 1). The signal processing unit SP constitutes, for example, an application specific integrated circuit (ASIC). The signal processing unit SP may include a CMOS circuit that converts the signal output from each semiconductor light detection element 6 into a digital pulse.

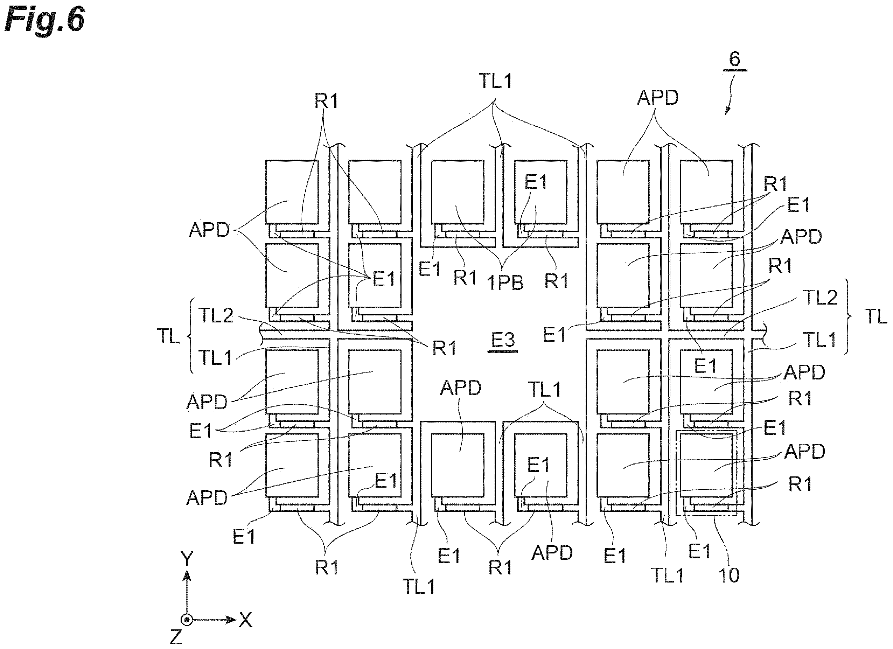

As shown in FIG. 6, in the semiconductor light detection element 6, the readout wiring TL includes a plurality of signal lines TL1 and a plurality of signal lines TL2. As an example, each signal line TL1 extends in the Y-axis direction between the avalanche photodiodes APD adjacent in the X-axis direction, and each signal line TL2 extends in the X-axis direction between the avalanche photodiodes APD adjacent in the Y-axis direction. The plurality of signal lines TL1 and the plurality of signal lines TL2 extend in a grid pattern to be connected to each other at intersection points and are electrically connected to the common electrode E3.

In each light detection unit 10, one end of the quenching resistor R1 is connected to an electrode E1, and the other end of the quenching resistor R1 is connected to the signal line TL1. That is, in each light detection unit 10, one end of the quenching resistor R1 is electrically connected to the anode of the avalanche photodiode APD via the electrode E1, and the other end of the quenching resistor R1 is electrically connected to the common electrode E3 via a readout wiring TL.

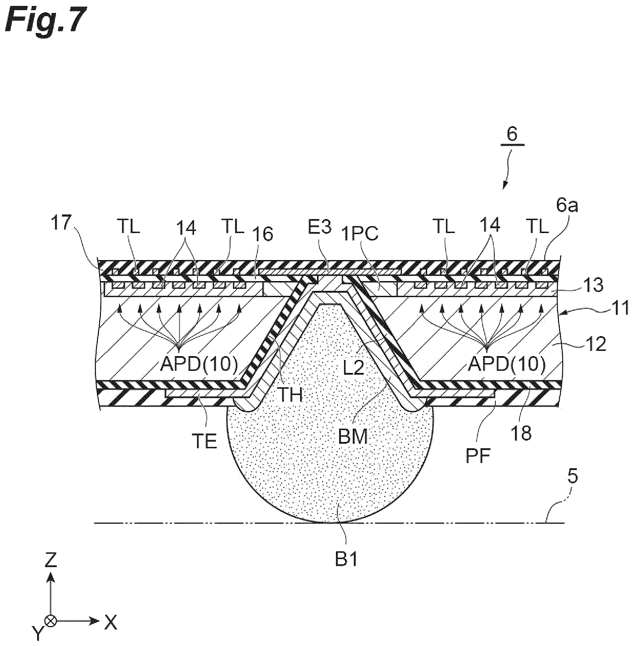

As shown in FIG. 7, the semiconductor light detection element 6 has a semiconductor layer 11. The semiconductor layer 11 includes an N-type (first conductivity type) semiconductor region (a first semiconductor region) 12, a P-type (second conductivity type) semiconductor region (a second semiconductor region) 13, and a plurality of P-type semiconductor regions (second semiconductor regions) 14. The semiconductor region 13 is formed on a surface of the semiconductor region 12 on the light incidence side. The plurality of semiconductor regions 14 are formed in the semiconductor region 13 along a surface of the semiconductor region 13 on the light incidence side. The impurity concentration of each semiconductor region 14 is higher than that of the semiconductor region 13.

In the semiconductor light detection element 6, one avalanche photodiode APD is constituted by one semiconductor region 14 and regions of the semiconductor regions 12 and 13 overlapping the one semiconductor region 14 in the Z-axis direction. That is, each avalanche photodiode APD includes the N-type semiconductor region 12 and the P-type semiconductor regions 13 and 14 forming PN junctions with the N-type semiconductor region 12. The P-type semiconductor regions 13 and 14 are located on a side of a surface 6a of the semiconductor light detection element 6 with respect to the N-type semiconductor region 12. The surface 6a is a surface of the semiconductor light detection element 6 on the light incidence side.

An insulating layer 16 is formed on a surface of the semiconductor region 13 on the light incidence side. The common electrode E3 and the readout wiring TL are disposed on the insulating layer 16. The common electrode E3 and the readout wiring TL are covered with an insulating layer 17. In the semiconductor light detection element 6, a surface of the insulating layer 17 on the light incidence side corresponds to the surface 6a of the semiconductor light detection element 6. In each light detection unit 10, one end of the quenching resistor R1 (see FIG. 6) is electrically connected to the semiconductor regions 13 and 14 of the avalanche photodiode APD, and the other end of the quenching resistor R1 is electrically connected to the readout wiring TL.

A through hole TH is formed in the semiconductor layer 11. An insulating layer 18 is formed on an inner surface of the through hole TH and a surface of the semiconductor region 12 on a side opposite to the light incidence side. A through electrode TE is disposed on the inner surface of the through hole TH via the insulating layer 18. The through electrode TE is connected to the common electrode E3 at an opening of the through hole TH on the light incidence side. A bump electrode B1 is disposed on the through electrode TE via an under bump metal BM. The through electrode TE and the insulating layer 18 are covered with a passivation film PF. An N-type semiconductor region 1PC is formed in a region surrounding the through hole TH on the surface of the semiconductor region 12 on the light incidence side. The semiconductor region 1PC prevents the PN junction formed by the N-type semiconductor region 12 and the P-type semiconductor regions 13 and 14 from reaching the through hole TH.

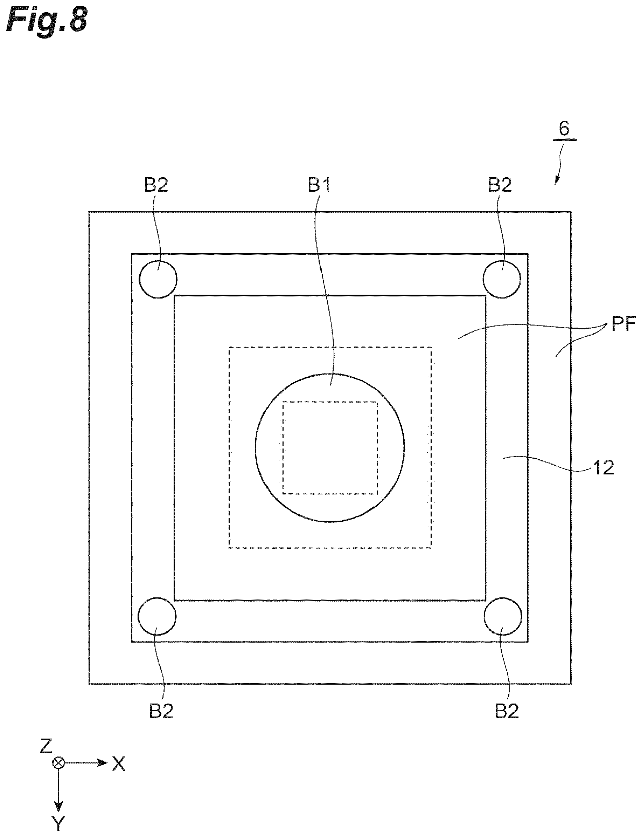

As shown in FIG. 8, a groove is formed in the passivation film PF to surround the through hole TH when viewed in the Z-axis direction, and the semiconductor region 12 is exposed in the groove. A plurality of bump electrodes B2 are disposed on the semiconductor region 12 exposed in the groove. The bump electrode B1 and the plurality of bump electrodes B2 are electrically and physically connected to the wiring substrate 5 disposed on a side opposite to the plurality of metalenses 8 with respect to the semiconductor light detection element 6. That is, the semiconductor light detection element 6 is electrically and physically connected to the wiring substrate 5.

In the semiconductor light detection element 6 configured as described above, the avalanche photodiode APD in each light detection unit 10 is operated in the Geiger mode. In this state, when light is incident on the semiconductor region 12 from a side of the surface 6a, photoelectric conversion occurs in the semiconductor region 12, and photoelectrons (electric charges) are generated in the semiconductor region 12. In the avalanche photodiode APD in which photoelectrons are generated, avalanche multiplication occurs in the semiconductor region 13, and an amplified electron group (electric charges) is collected in the common electrode E3 via the semiconductor region 14 and the quenching resistor R1. The electric charges collected in the common electrode E3 from each light detection unit 10 are input to the signal processing unit SP (see FIG. 5) of the wiring substrate 20 as a signal.

The semiconductor layer 11 is made of Si, for example. In the semiconductor layer 11, the P-type impurities are, for example, Group 3 elements such as B, and the N-type impurities are, for example, Group 5 elements such as N, P, and As. A method of adding these impurities is, for example, a diffusion method or an ion injection method. Each insulating layer 16, 17, or 18 is made of, for example, $SiO_2$ or SiN. A method of forming each insulating layer 16, 17, or 18 is, for example, a thermal oxidation method or a sputtering method. The electrodes E1 and E3 and the through electrode TE are made of a metal such as aluminum, for example. A method of forming the electrodes E1 and E3 and the through electrode TE is, for example, a sputtering method. The resistivity of the quenching resistor R1 is higher than the resistivity of the electrode E1 and the resistivity of the common electrode E3. The quenching resistor R1 is made of polysilicon, for example. A method of forming the quenching resistor R1 is, for example, a chemical vapor deposition (CVD) method. The material of the quenching resistor R1 may be, for example, SiCr, NiCr, TaNi, FeCr, or the like.

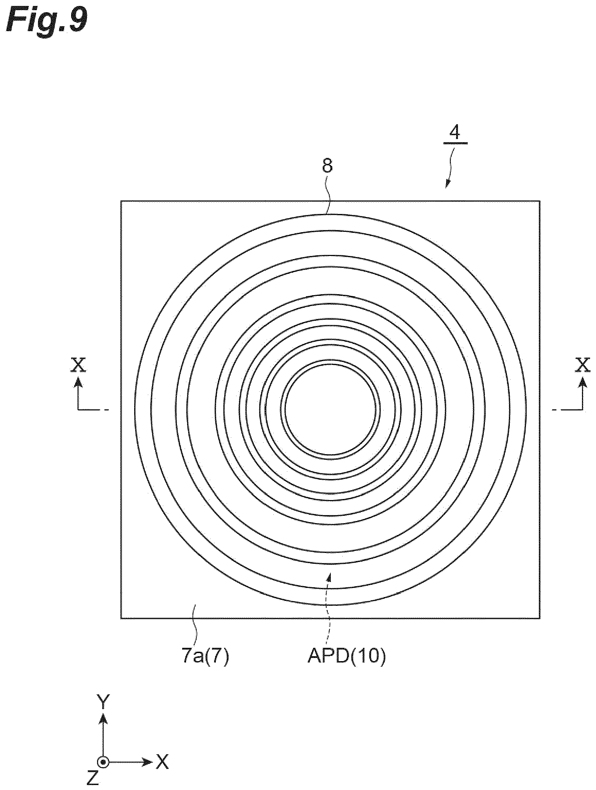

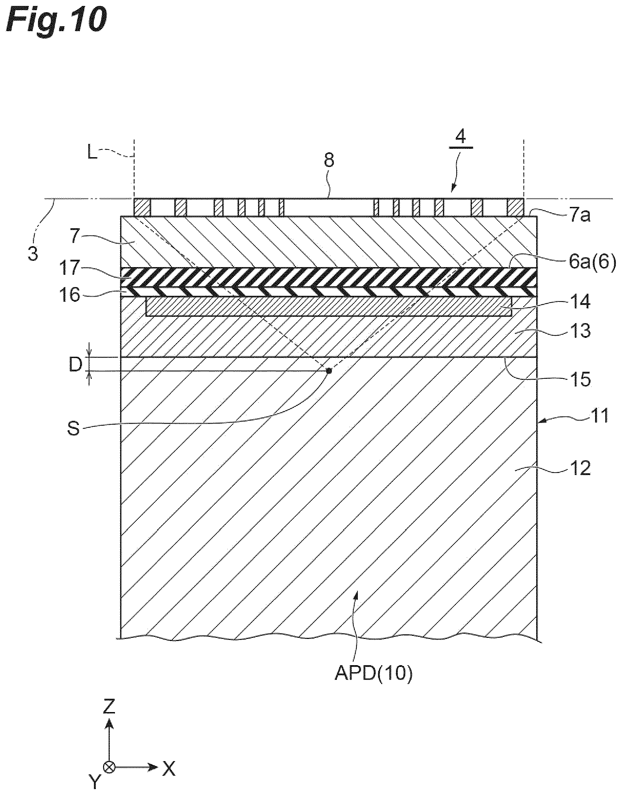

As shown in FIGS. 3, 9 and 10, the plurality of metalenses 8 are provided on the light transmitting substrate 7. The plurality of metalenses 8 are disposed on the surface 6a of the semiconductor light detection element 6 via the light transmitting substrate 7. Each metalens 8 is two-dimensionally disposed to overlap each light detection unit 10 when viewed in the Z-axis direction (a direction intersecting with the surface 6a). That is, one metalens 8 corresponds to one light detection unit 10 (faces in the Z-axis direction). In FIGS. 9 and 10, only a portion of the light detector 4 corresponding to one light detection unit 10 is illustrated.

The plurality of metalenses 8 are metasurface lenses formed on a surface 7a of the light transmitting substrate 7. The light transmitting substrate 7 having the plurality of metalenses 8 formed on the surface 7a is bonded to the surface 6a of the semiconductor light detection element 6 with a light transmitting adhesive. The light transmitting substrate 7 is made of, for example, $SiO_2$, GaAs, GaP, Si, SiC, or the like. The plurality of metalenses 8 are made of a metalens material such as a-Si, $HfO_2$, $Nb_2O_5$, or $TiO_2$, for example. As an example, the plurality of metalenses 8 are formed on the surface 7a of the light transmitting substrate 7 as follows. That is, a film made of a metalens material is formed on the surface of the light transmitting substrate 7, an EB mask is formed on the film by an EB lithography method, and the film is subjected to an etching process, and thus the plurality of metalenses 8 are formed on the surface 7a of the light transmitting substrate 7.

The metalens 8 is configured on the basis of the phase design of Fresnel lens, for example. The outer diameter of the metalens 8 is, for example, about 75 $\mu$m. The outer diameter of the metalens 8 is designed to correspond to the size of the light detection unit 10. The thickness of the metalens 8 in the Z-axis direction is, for example, about 500 nm. A single period of the metalens 8 is equal to or less than the wavelength of the light L, for example, about 250 nm. The thickness of the light transmitting substrate 7 is determined such that a convergence spot S, which will be described later, is located at a desired position. The thickness of the light transmitting substrate 7 is, for example, about several tens of $\mu$m.

As shown in FIG. 10, in the metalenses 8 and the light detection unit 10 facing each other in the Z-axis direction, the metalenses 8 converge the light L such that the convergence spot S is located at a position which is within the semiconductor region 12 and which is separated by a predetermined distance D from a boundary 15 between the semiconductor region 12 and the semiconductor region 13 in the Z-axis direction. The convergence spot S means a beam waist portion of the light L that has passed through the metalenses 8 (that is, a portion where the beam diameter of the light L is the smallest). The distance between the metalens 8 and the convergence spot S in the Z-axis direction is approximately 50 $\mu$m.

Here, when a wavelength of the light L is defined as $\lambda$ ($\mu$m), each metalens 8 has a numerical aperture of $((0.61)^2\pi\lambda)^{1/2}$ or more. In other words, when the numerical aperture of the metalens 8 is defined as NA, the following expression (1) is satisfied.

$$NA \geq ((0.61)^2\pi\lambda)^{1/2} \ ... \ (1)$$

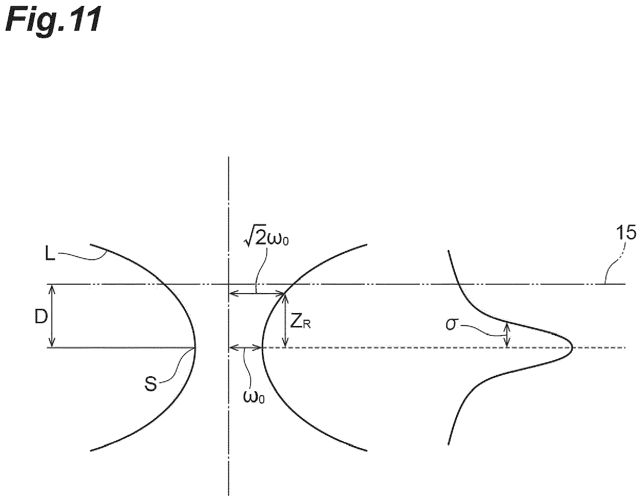

Further, as shown in FIG. 11, when Rayleigh length of the light L converged by the metalenses 8 is defined as $Z_R$ ($\mu$m) and a beam waist is defined as $\omega_0$ ($\mu$m), the following expressions (2) and (3) are satisfied.

$$Z_R = \pi\omega_0^2/\lambda \ ... \ (2)$$

$$\omega_0 = 0.61\lambda/NA \ ... \ (3)$$

The following expression (4) is derived from the above expressions (1), (2) and (3). As shown in FIG. 10, since the light L is incident on the semiconductor layer 11 through the light transmitting substrate 7 and the insulating layers 16 and 17, strictly speaking, the effect of aberration occurs, but the expression (4) means that the Rayleigh length of the light L converged by the metalenses 8 is sufficiently shortened to about 1 $\mu$m or less.

$$Z_R \leq 1 \ \mu m \ ... \ (4)$$

Further, when a thickness of the semiconductor region 12 in the Z-axis direction is defined as t, the predetermined distance D is less than t/2. From another point of view, the predetermined distance D is 0 $\mu$m or more and 3 $\mu$m or less. This means that at least a region of the light L corresponding to the Rayleigh length $Z_R$ on the downstream side of the convergence spot S is located within the semiconductor region 12 in a case where the above expression (4) is satisfied (see FIGS. 10 and 11). This is because the convergence spot S is located on the boundary 15 between the semiconductor regions 12 and 13 in a case where the predetermined distance D is 0 $\mu$m. From another point of view, when a standard deviation of a Gaussian function in a case where a beam profile of the convergence spot S in the Z-axis direction is approximated to the Gaussian function is defined as $\sigma$, the predetermined distance D is 0$\sigma$ or more and 3$\sigma$ or less. This means that at least a region of the light L corresponding to the standard deviation $\sigma$ on the downstream side of the convergence spot S is located within the semiconductor region 12 (see FIGS. 10 and 11). This is because the convergence spot S is located on the boundary 15 between the semiconductor regions 12 and 13 in a case where the predetermined distance D is 0$\sigma$. As a result, in the light detector 4, photoelectric conversion occurs at a portion in the vicinity of the semiconductor region 13 in the semiconductor region 12 in each light detection unit 10, and electric charges are likely to be generated in this region. The fact that the predetermined distance D is 1 $\mu$m or more in a case where the above expression (4) is satisfied means that a region of the light L corresponding to the Rayleigh length $Z_R$ on the upstream side of the convergence spot S is located within the semiconductor region 12. Similarly, the fact that the predetermined distance D is $\sigma$ or more in a case where the above expression (4) is satisfied means that a region of the light L corresponding to the Rayleigh length $Z_R$ on the upstream side of the convergence spot S is located within the semiconductor region 12.

As described above, in the light detector 4, the metalenses 8 converge the light L such that the convergence spot S is located at a position which is within the semiconductor region 12 of each light detection unit 10 and which is separated by a predetermined distance D from a boundary 15 between the semiconductor region 12 and the semiconductor region 13 in the Z-axis direction. As a result, the depth of the portion in the semiconductor region 12 where electric charges are generated is uniformized among the plurality of light detection units 10. Therefore, according to the light detector 4, the response time (a time from the generation of the electric charges to the detection of the electric charges) can be sufficiently uniformized.

The plurality of metalenses 8 have an excellent effect as compared with, for example, a resin-made microlens array. That is, since the plurality of metalenses 8 are formed of an inorganic substance with a thickness equal to or less than the wavelength of the light L, the plurality of metalenses 8 are physically and chemically stable. In particular, since the scintillator 3 is disposed on the plurality of metalenses 8, the metalenses 8 that are difficult to be deformed by the weight of the scintillator 3 are extremely effective in reliably locating the convergence spot S at a position separated by the predetermined distance D from the boundary 15 between the semiconductor region 12 and the semiconductor region 13. The metalens 8 also has the same effect in that it is difficult to deform due to heat. Further, according to the metalens 8, it is possible to design the phase of the lens by controlling a two-dimensional shape, and it is also possible to realize a high numerical aperture.

In the light detector 4, when a thickness of the semiconductor region 12 in the Z-axis direction is defined as t, the predetermined distance D is less than t/2. As a result, the portion where the electric charges are generated in the semiconductor region 12 is sufficiently close to the semiconductor region 13, and thus it is possible to more reliably curb variations in response time.

In the light detector 4, when a standard deviation of the Gaussian function in a case where a beam profile of the convergence spot S in the Z-axis direction is approximated to the Gaussian function is defined as $\sigma$, the predetermined distance D is 0$\sigma$ or more and 3$\sigma$ or less. As a result, the portion where the electric charges are generated in the semiconductor region 12 is sufficiently close to the semiconductor region 13, and thus it is possible to more reliably curb variations in response time.

In the light detector 4, when a wavelength of the light L is defined as $\lambda$ ($\mu$m), each metalens 8 has a numerical aperture of $((0.61)^2\pi\lambda)^{1/2}$ or more. As a result, the Rayleigh length of the light L converged by the metalenses 8 is sufficiently shortened to about 1 $\mu$m or less, and thus it is possible to more reliably curb variations in response time.

In the light detector 4, the predetermined distance D is 0 $\mu$m or more and 3 $\mu$m or less. As a result, the portion where the electric charges are generated in the semiconductor region 12 is sufficiently close to the semiconductor region 13, and thus it is possible to more reliably curb variations in response time.

In the light detector 4, the plurality of metalenses 8 are provided on the light transmitting substrate 7 and disposed on the surface 6a of the semiconductor light detection element 6 via the light transmitting substrate 7. As a result, in manufacturing the light detector 4, for example, it is possible to improve the yield of the light detector 4 by bonding the light transmitting substrate 7 on which the plurality of metalenses 8 are formed to the surface 6a of the semiconductor light detection element 6. In addition, it is possible to make the light transmitting substrate 7 function as a spacer between the metalenses 8 and the light detection unit 10, and it is possible to reliably locate the convergence spot S at a position separated by the predetermined distance D from the boundary 15 between the semiconductor region 12 and the semiconductor region 13.

In the light detector 4, the wiring substrate 5 is disposed on a side opposite to the plurality of metalenses 8 with respect to the semiconductor light detection element 6, and the semiconductor light detection element 6 is electrically and physically connected to the wiring substrate 5. As a result, an external wiring can be connected to the wiring substrate 5 from a side opposite to the plurality of metalenses 8.

According to the radiation detector 2, it is possible to sufficiently uniformize the response time in the light detector 4 for the reasons described above.

According to the PET device 1, it is possible to sufficiently uniformize the response time in the plurality of light detectors 4 for the reasons described above, and thus it is possible to acquire a high-quality image.

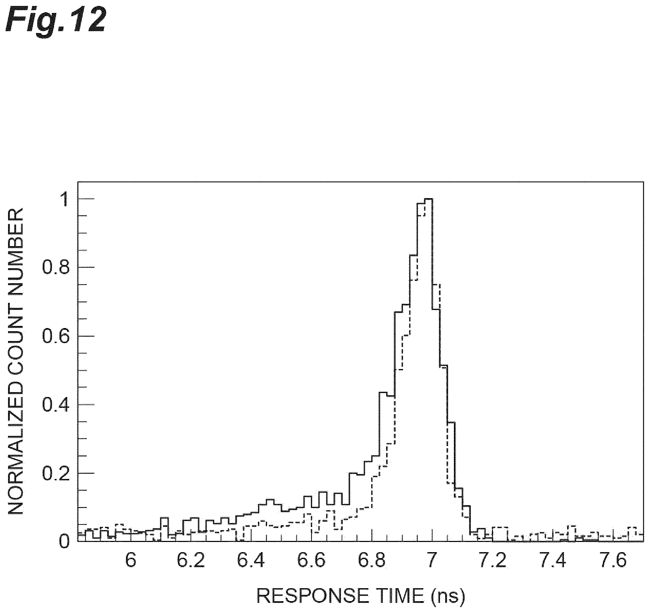

FIG. 12 is a graph showing time resolution of light detectors of an example and a comparative example. In the graph shown in FIG. 12, a horizontal axis indicates the response time in the light detectors of the example and the comparative example, and a vertical axis indicates a normalized count number (a normalized count number of the detection signal) in the light detectors of the example and the comparative example. In the graph shown in FIG. 12, a dashed line indicates the time resolution of the light detector of the example, and a solid line indicates the time resolution of the light detector of the comparative example. As the light detector of the example, a light detector having the same configuration as the light detector 4 of the above embodiment was used. As the light detector of the comparative example, a light detector having a configuration in which the light transmitting substrate 7 and the plurality of metalens 8 are removed from the light detector 4 of the above embodiment was used. As shown in FIG. 12, the half width of the time resolution in the light detector of the example was smaller than the half width of the time resolution in the light detector of the comparative example.

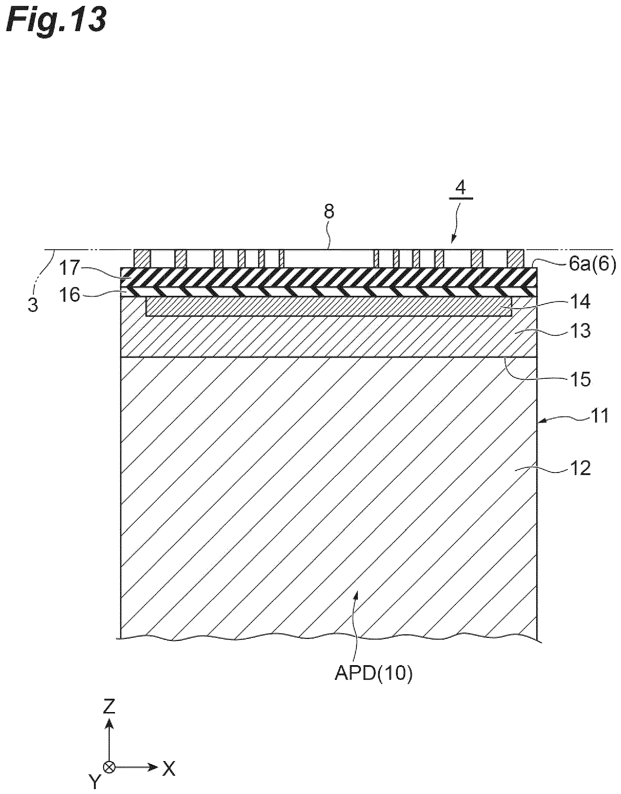

The present disclosure is not limited to the above embodiment. For example, as shown in FIG. 13, the plurality of metalens 8 may be disposed directly on the surface 6a of the semiconductor light detection element 6. Moreover, the plurality of metalenses 8 may be configured by forming grooves on the surface 7a of the light transmitting substrate 7. Further, the semiconductor light detection element 6 as a SiPM having a plurality of SPADs (light detection units 10) may have other configurations such as a configuration in which N-type and P-type are reversed. Further, a detection target of the radiation detector 2 is not limited to an annihilation $\gamma$ ray and may be another radiation such as an X ray. In the radiation detector 2, the light emitting body that emits light upon incidence of radiation is not limited to the scintillator 3 and may be another light emitting body such as Cherenkov radiator.

## Reference Signs List

1 PET device
2 Radiation detector
3 Scintillator (light emitting body)
4 Light detector
5 Wiring substrate
6 Semiconductor light detection element
6a Surface
7 Light transmitting substrate
8 Metalens
10 Light detection unit
12 Semiconductor region (first semiconductor region)
13, 14 Semiconductor region (second semiconductor region)
15 Boundary
APD Avalanche photodiode
R1 Quenching resistor
TL Readout wiring
L Light
S Convergence spot

## Claims

1. A light detector comprising:

   a semiconductor light detection element having a plurality of light detection units disposed two-dimensionally and readout wirings; and
   a plurality of metalenses disposed on a surface of the semiconductor light detection element,
   wherein each of the plurality of light detection units has
   an avalanche photodiode including a first semiconductor region of a first conductivity type and a second semiconductor region of a second conductivity type, the second semiconductor region located on a side of the surface with respect to the first semiconductor region and forming a PN junction with the first semiconductor region, and
   a quenching resistor including one end electrically connected to the second semiconductor region and another end electrically connected to the readout wiring, and
   wherein the plurality of metalenses are disposed two-dimensionally to overlap the plurality of light detection units when seen in a direction intersecting with the surface, and converge light such that a convergence spot is located at a position which is within the first semiconductor region of each of the plurality of light detection units and which is separated by a predetermined distance from a boundary between the first semiconductor region and the second semiconductor region in the direction intersecting with the surface.

2. The light detector according to claim 1, wherein, when a thickness of the first semiconductor region in the direction intersecting with the surface is defined as t, the predetermined distance is less than t/2.

3. The light detector according to claim 1 or 2, wherein, when a standard deviation of a Gaussian function in a case where a beam profile of the convergence spot in the direction intersecting with the surface is approximated to the Gaussian function is defined as $\sigma$, the predetermined distance is $0\sigma$ or more and $3\sigma$ or less.

4. The light detector according to any one of claims 1 to 3, wherein, when a wavelength of the light is defined as $\lambda$ ($\mu$m), each of the plurality of metalenses has a numerical aperture of $((0.61)^2\pi\lambda)^{1/2}$ or more.

5. The light detector according to claim 4, wherein the predetermined distance is $0$ $\mu$m or more and $3$ $\mu$m or less.

6. The light detector according to any one of claims 1 to 5, further comprising:

   a light transmitting substrate provided with the plurality of metalenses,
   wherein the plurality of metalenses are disposed on the surface of the semiconductor light detection element via the light transmitting substrate.

7. The light detector according to any one of claims 1 to 6, further comprising:

   a wiring substrate disposed on a side opposite to the plurality of metalenses with respect to the semiconductor light detection element,
   wherein the semiconductor light detection element is electrically and physically connected to the wiring substrate.

8. A radiation detector comprising:

   a light emitting body configured to emit light upon incidence of radiation; and
   the light detector according to any one of claims 1 to 7,
   wherein the light emitting body is disposed on a side opposite to the semiconductor light detection element with respect to the plurality of metalenses.

9. A PET device comprising:

   a plurality of radiation detectors disposed in an annular shape,

wherein each of the plurality of radiation detectors is the radiation detector according to claim 8.

# Fig.1

**Fig.2**

# *Fig.3*

*Fig.4*

6

E3

10

Y

Z X

*Fig.5*

**Fig.6**

EP 4 212 914 A1

# Fig.7

# *Fig.8*

*Fig.9*

# Fig.10

# Fig.11

*Fig.12*

*Fig.13*

APD(10)

## INTERNATIONAL SEARCH REPORT

| | International application No. |
|---|---|
| | **PCT/JP2021/032498** |

| | |
|---|---|
| **A.** | **CLASSIFICATION OF SUBJECT MATTER** |

*G01T 1/161*(2006.01)i; *G01T 1/20*(2006.01)i; *H01L 31/0232*(2014.01)i; *H01L 31/107*(2006.01)i
FI:  H01L31/02 D; G01T1/20 E; G01T1/20 G; G01T1/20 C; H01L31/10 B; G01T1/161 A; G01T1/161 C

According to International Patent Classification (IPC) or to both national classification and IPC

| | |
|---|---|
| **B.** | **FIELDS SEARCHED** |

Minimum documentation searched (classification system followed by classification symbols)

H01L31/00-31/0392; H01L31/08-31/119; H01L31/18-31/20

Documentation searched other than minimum documentation to the extent that such documents are included in the fields searched

Published examined utility model applications of Japan 1922-1996
Published unexamined utility model applications of Japan 1971-2021
Registered utility model specifications of Japan 1996-2021
Published registered utility model applications of Japan 1994-2021

Electronic data base consulted during the international search (name of data base and, where practicable, search terms used)

| | |
|---|---|
| **C.** | **DOCUMENTS CONSIDERED TO BE RELEVANT** |

| Category* | Citation of document, with indication, where appropriate, of the relevant passages | Relevant to claim No. |
|---|---|---|
| Y | JP 2020-17943 A (CANON INC.) 30 January 2020 (2020-01-30) paragraphs [0008], [0013], [0014], [0018], [0019], [0023], [0025]-[0028], [0036], [0037], [0045], [0046], [0064], [0097], fig. 1-5, 12 | 1-9 |
| Y | JP 2018-46395 A (HAMAMATSU PHOTONICS KK) 22 March 2018 (2018-03-22) paragraphs [0019], [0020], fig. 1 | 1-9 |
| Y | JP 2016-192551 A (HAMAMATSU PHOTONICS KK) 10 November 2016 (2016-11-10) paragraphs [0030], [0031], [0052], [0065], [0070], [0072], [0074], [0082], [0083], [0088], [0090], fig. 2, 4, 10-11, 14 | 1-9 |
| A | JP 2015-119093 A (HAMAMATSU PHOTONICS KABUSHIKI KAISHA) 25 June 2015 (2015-06-25) entire text, all drawings | 1-9 |
| A | JP 2015-179087 A (TOSHIBA CORP.) 08 October 2015 (2015-10-08) entire text, all drawings | 1-9 |
| A | US 2019/0099138 A1 (GENERAL ELECTRIC CO.) 04 April 2019 (2019-04-04) entire text, all drawings | 1-9 |

☐ Further documents are listed in the continuation of Box C.  ☑ See patent family annex.

| | |
|---|---|
| * Special categories of cited documents: | "T" later document published after the international filing date or priority date and not in conflict with the application but cited to understand the principle or theory underlying the invention |
| "A" document defining the general state of the art which is not considered to be of particular relevance | |
| "E" earlier application or patent but published on or after the international filing date | "X" document of particular relevance; the claimed invention cannot be considered novel or cannot be considered to involve an inventive step when the document is taken alone |
| "L" document which may throw doubts on priority claim(s) or which is cited to establish the publication date of another citation or other special reason (as specified) | "Y" document of particular relevance; the claimed invention cannot be considered to involve an inventive step when the document is combined with one or more other such documents, such combination being obvious to a person skilled in the art |
| "O" document referring to an oral disclosure, use, exhibition or other means | |
| "P" document published prior to the international filing date but later than the priority date claimed | "&" document member of the same patent family |

| Date of the actual completion of the international search | Date of mailing of the international search report |
|---|---|
| **28 September 2021** | **12 October 2021** |

| Name and mailing address of the ISA/JP | Authorized officer |
|---|---|
| **Japan Patent Office (ISA/JP)** **3-4-3 Kasumigaseki, Chiyoda-ku, Tokyo 100-8915 Japan** | |
| | Telephone No. |

Form PCT/ISA/210 (second sheet) (January 2015)

**INTERNATIONAL SEARCH REPORT**
Information on patent family members

International application No.

**PCT/JP2021/032498**

| Patent document cited in search report | | | Publication date (day/month/year) | Patent family member(s) | | | Publication date (day/month/year) |
|---|---|---|---|---|---|---|---|
| JP | 2020-17943 | A | 30 January 2020 | US paragraphs [0007], [0025]-[0027], [0032], [0033], [0037], [0039]-[0041], [0050], [0051], [0059]-[0061], [0079], [0114], fig. 1-5, 12 CN | 2020/0021767 110719419 | A1 A | |
| JP | 2018-46395 | A | 22 March 2018 | US paragraphs [0049], [0050], fig. 1 | 2018/0074227 | A1 | |
| JP | 2016-192551 | A | 10 November 2016 | (Family: none) | | | |
| JP | 2015-119093 | A | 25 June 2015 | US entire text, all drawings EP CN | 2016/0322417 3086375 105830232 | A1 A1 A | |
| JP | 2015-179087 | A | 08 October 2015 | (Family: none) | | | |
| US | 2019/0099138 | A1 | 04 April 2019 | CN | 109613588 | A | |

Form PCT/ISA/210 (patent family annex) (January 2015)

24

**EP 4 212 914 A1**

**Patent documents cited in the description**

- JP 2020115575 A